(19)

Europäisches
Patentamt
European
Patent Office
Office européen
des brevets

(11) **EP 4 023 748 A1**

(12) **EUROPEAN PATENT APPLICATION**
published in accordance with Art. 153(4) EPC

(43) Date of publication:
**06.07.2022 Bulletin 2022/27**

(21) Application number: **20858676.8**

(22) Date of filing: **28.08.2020**

(51) International Patent Classification (IPC):
**C12N 5/0793** (2010.01)    **C12N 5/10** (2006.01)
**C12N 15/113** (2010.01)

(52) Cooperative Patent Classification (CPC):
**C12N 5/10; C12N 15/113**

(86) International application number:
**PCT/JP2020/032606**

(87) International publication number:
**WO 2021/039965 (04.03.2021 Gazette 2021/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.08.2019 JP 2019157194**

(71) Applicant: **Keio University
Tokyo, 108-8345 (JP)**

(72) Inventors:
- **ISHIKAWA, Mitsuru
  Tokyo 160-8582 (JP)**
- **OKANO, Hideyuki
  Tokyo 160-8582 (JP)**

(74) Representative: **Cabinet Becker et Associés
25, rue Louis le Grand
75002 Paris (FR)**

(54) **METHOD FOR PRODUCING PARVALBUMIN-POSITIVE NERVE CELLS, CELL, AND DIFFERENTIATION INDUCER**

(57)    A production method for parvalbumin-positive nerve cells includes: an expression induction step of inducing expression of Ascl1 gene, Dlx2 gene, and MEF2C gene in a cell, and a differentiation step of culturing the cell after the expression induction to differentiate the cells into parvalbumin-positive nerve cell; a cell into which Ascl1 gene, Dlx2 gene, and MEF2C gene are introduced in an expressible manner; and a differentiation inducer for inducing differentiation of a cell into a parvalbumin-positive nerve cell, including Ascl1 gene, Dlx2 gene, and MEF2C gene, or gene products thereof, as an active ingredient.

EP 4 023 748 A1

**Description**

**TECHNICAL FIELD**

The present invention relates to a production method for parvalbumin-positive nerve cells, a cell, and a differentiation inducer.

Priority is claimed on Japanese Patent Application No. 2019-157194, filed August 29, 2019, the content of which is incorporated herein by reference.

**BACKGROUND ART**

Parvalbumin-positive nerve cells are one of the most important nerve cells involved in diseases of the brain and nervous system and it is thought that a decrease in the abundance or function of parvalbumin-positive nerve cells causes various psycho-neurological diseases and neurodevelopmental disorders (Non Patent Document 1, Non Patent Document 2). For this reason, much attention has been paid to the elucidation of the functions thereof over the years and, particularly in recent years, there has been a strong demand for techniques for creating parvalbumin-positive nerve cells from pluripotent stem cells such as human iPS cells.

However, although there have been reports of specific methods for inducing differentiation of inhibitory nerve cells, including parvalbumin-positive nerve cells and the like in a portion thereof, the efficiency of inducing the parvalbumin-positive nerve cells themselves was extremely low (Non Patent Document 3). In addition, Non Patent Document 4 reports a method having the highest efficiency for the ratio of appearance of parvalbumin-positive nerve cells among the induction methods of the related art; however, this method requires approximately 80 days to produce parvalbumin-positive nerve cells through a plurality of differentiation induction steps, after which the positive ratio of the produced parvalbumin-positive nerve cells is approximately 20% to 30%.

Citation List

Non Patent Documents

[Non Patent Document 1]
Lewis DA et al., Trends Neurosci. 2012 Jan; 35(1):57-67.
[Non Patent Document 2]
Rodriguez RA et al., Front Mol Neurosci. 2018 Apr 24; 11:132.
[Non Patent Document 3]
Yang N. et al., Nat Methods. 2017 Jun; 14(6): 621-628.
[Non Patent Document 4]
Yuan F. et al., eLIFE. 2018 Sep 25;7. pii: e37382.

**DISCLOSURE OF INVENTION**

Technical Problem

An object of the present invention is to provide a production method for parvalbumin-positive nerve cells with high efficiency in a short period of time with few differentiation induction steps, cells able to be induced into the parvalbumin-positive nerve cells, and a differentiation inducer for inducing differentiation into the parvalbumin-positive nerve cells.

Solution to Problem

The present invention includes the following aspects.

[1] A production method for parvalbumin-positive nerve cells, including an expression induction step of inducing expression of Ascl1 gene, Dlx2 gene, and MEF2C gene in a cell, and a differentiation step of culturing the cell after the expression induction to differentiate the cells into parvalbumin-positive nerve cell.
[2] The production method for parvalbumin-positive nerve cells according to [1], in which the expression induction step includes a gene introduction step of introducing Ascl1 gene and Dlx2 gene into the cell in an expressible manner.
[3] The production method for parvalbumin-positive nerve cells according to [2], in which the expression induction step further includes a gene introduction step of introducing MEF2C gene in an expressible manner.

[4] The production method according to any one of [1] to [3], in which the Ascl1 gene, the Dlx2 gene, and the MEF2C gene are tetracycline-regulated (Tet-ON).

[5] The production method according to any one of [1] to [4], in which the expression induction step includes a step of simultaneously inducing expression of at least one selected from the group consisting of microRNA-9/9* (miRNA-9/9*), microRNA-124 (miRNA-124), and BclxL gene.

[6] The production method according to [5], in which the expression induction step includes a gene introduction step of introducing at least one selected from the group consisting of microRNA-9/9* (miRNA-9/9*), microRNA-124 (miRNA-124), and BclxL gene in an expressible manner.

[7] The production method according to [5] or [6], in which the microRNA-9/9* (miRNA-9/9*), the microRNA-124 (miRNA-124), and the BclxL gene are tetracycline-regulated (Tet-ON).

[8] The production method according to any one of [1] to [7], in which the cell is a fibroblast or a pluripotent stem cell.

[9] The production method according to any one of [1] to [8], in which the expression induction step is performed for 1 to 5 days.

[10] The production method according to any one of [1] to [9], in which the differentiation step is performed for 10 days or more.

[11] A cell into which Ascl1 gene, Dlx2 gene, and MEF2C gene are introduced in an expressible manner.

[12] The cell according to [11], in which the Ascl1 gene, the Dlx2 gene, and the MEF2C gene are tetracycline-regulated (Tet-ON).

[13] The cell according to [11] or [12], in which at least one selected from the group consisting of microRNA-9/9* (miRNA-9/9*), microRNA-124 (miRNA-124), and BclxL gene is further introduced in an expressible manner.

[14] The cell according to [13], in which the microRNA-9/9* (miRNA-9/9*), the microRNA-124 (miRNA-124), and the BclxL gene are tetracycline-regulated (Tet-ON).

[15] The cell according to any one of [11] to [14], in which the cell is a fibroblast or a pluripotent stem cell.

[16] A differentiation inducer for inducing differentiation of a cell into a parvalbumin-positive nerve cell, the differentiation inducer including Ascl1 gene, Dlx2 gene, and MEF2C gene, or gene products thereof, as an active ingredient.

[17] The differentiation inducer according to [16], in which the Ascl1 gene, the Dlx2 gene, and the MEF2C gene are tetracycline-regulated (Tet-ON).

[18] The differentiation inducer according to [16] or [17], further including at least one selected from the group consisting of microRNA-9/9* (miRNA-9/9*), microRNA-124 (miRNA-124), and BclxL gene, or gene products thereof, as an active ingredient.

[19] The differentiation inducer according to [18], in which the microRNA-9/9* (miRNA-9/9*), the microRNA-124 (miRNA-124), and the BclxL gene are tetracycline-regulated (Tet-ON).

[20] The differentiation inducer according to any one of [16] to [19], in which the cell is a fibroblast or a pluripotent stem cell.

Advantageous Effects of Invention

According to the present invention, it is possible to provide a production method for parvalbumin-positive nerve cells with high efficiency in a short period of time with few differentiation induction steps, cells able to be induced into the parvalbumin-positive nerve cells, and a differentiation inducer for inducing differentiation into the parvalbumin-positive nerve cells.

**BRIEF DESCRIPTION OF DRAWINGS**

Fig. 1A shows the time course of the introduction of a piggyBac vector into human pluripotent stem cells and cloning by drug selection.

Fig. 1B shows the time course of lentiviral vector infection into human pluripotent stem cells and Tet-driven neural induction by doxycycline. In the figure, MC indicates medium change, Neurobasal Plus B27 Plus indicates a medium for nerve cells, and Dox indicates doxycycline.

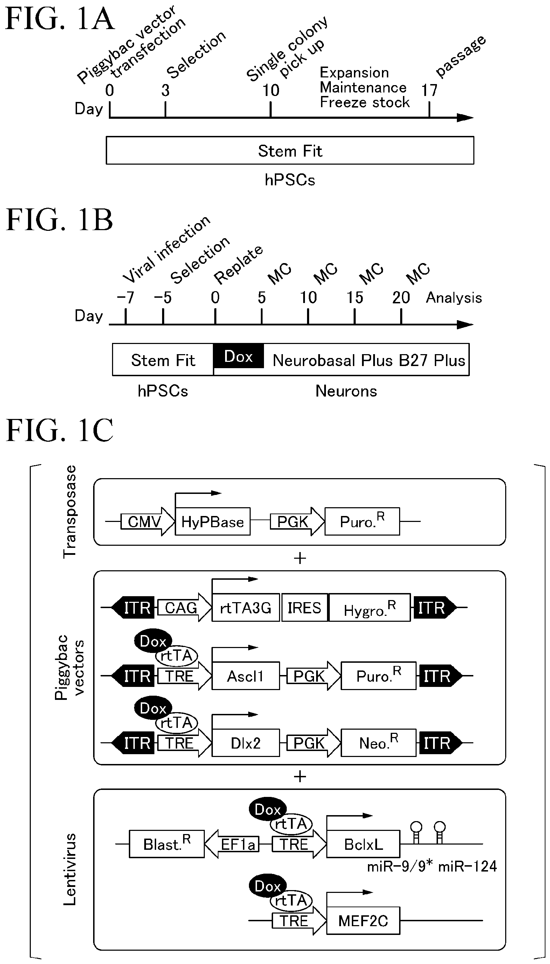

Fig. 1C is a diagram showing plasmid vectors and lentiviral vectors used for gene introduction. In the figure, CMV, PGK, CAG, and EF1a represent constant expression gene promoters. Puro.R represents a puromycin resistance gene, Hygro.R represents a hygromycin resistance gene, Neo.R represents a G418 resistance gene, Blast.R represents a blasticidin resistance gene, Tet represents a Tet operator DNA repeat element, HyPBase represents a transposase, rtTA3G represents a reverse tetracycline-regulated trans-activating factor, and ITR represents reverse-orientated repetitive sequences, respectively.

Fig. 2 is a diagram showing a scheme for the production of a parvalbumin gene (PVALB) reporter cell line.

Fig. 3 is a diagram showing immunostained images of cells after 20 days passed following neural induction (5 days of expression induction and 15 days of differentiation). In the figure, PV-GFP indicates an immunostained image with an Anti-PV antibody, GFP/Phase indicates a bright field image, Hoechst indicates a nuclear stained image, Anti-GFP indicates an immunostained image with an Anti-GFP antibody, respectively. + and - indicate gene introduction and no gene introduction, respectively.

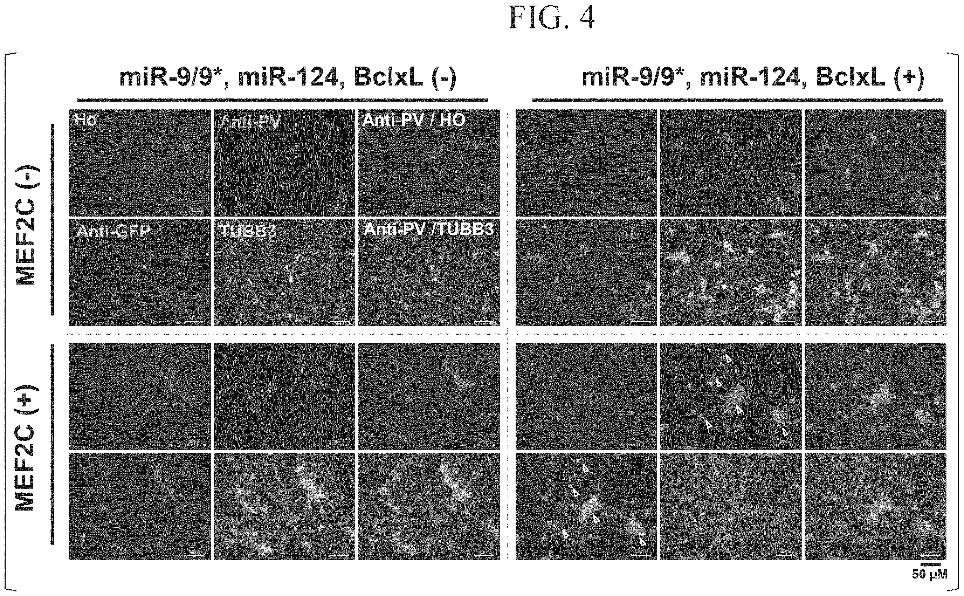

Fig. 4 is a diagram showing immunostained images of cells after 20 days following expression induction (5 days of expression induction and 15 days of differentiation). The left side of the diagram shows immunostained images of cells in a case where transient expression of miRNA-9/9*, miRNA-124, and BclxL gene was not performed while the right side of the diagram shows immunostained images of cells in a case where transient expression of miRNA-9/9*, miRNA-124, and BclxL gene was performed. + and - indicate gene introduction and no gene introduction, respectively. Arrows indicate typical cells of cells stained with Anti-PV antibodies and Anti-GFP antibodies.

Fig. 5 is a graph showing an analysis of the parvalbumin mRNA expression level by a quantitative PCR method. In the figure, + and - indicate gene introduction and no gene introduction, respectively.

## BEST MODE FOR CARRYING OUT THE INVENTION

[Production method for parvalbumin-positive nerve cells]

In one embodiment, the present invention provides a production method for parvalbumin-positive nerve cells (also referred to below as PV+ nerve cells), including an expression induction step of inducing expression of Ascl1 gene, Dlx2 gene, and MEF2C gene in cells, and a differentiation step of culturing the cells after expression induction to differentiate the cells into parvalbumin-positive nerve cells.

As described below in the Examples, according to the production method of the present embodiment, it is possible to provide a production method for PV+ nerve cells with high efficiency in a short period of time with few differentiation induction steps. Methods for inducing differentiation of PV+ nerve cells in the related art have many differentiation induction steps and the content of PV+ nerve cells is approximately 20% to 30% at most. Moreover, a long period of approximately 60 to 80 days was necessary to induce differentiation into PV+ nerve cells. In contrast, according to the method of the present embodiment, it is possible to produce a cell population in which 85% or more are PV+ nerve cells in 20 days with few differentiation induction steps.

The cells used in the production method of the present embodiment are not particularly limited as long as the cells are able to be induced into PV+ nerve cells and examples thereof include fibroblasts, mesenchymal stem cells, pluripotent stem cells, and the like, but fibroblasts and pluripotent stem cells are preferable.

In the present specification, examples of pluripotent stem cells include embryonic stem cells (ES cells), induced pluripotent stem cells (iPS cells), and the like. Pluripotent stem cells may be human-derived cells or may be cells derived from non-human animals such as mice, rats, pigs, goats, sheep, and monkeys.

In addition, the pluripotent stem cells described above may be induced pluripotent stem cells derived from healthy individuals or may be induced pluripotent stem cells derived from patients with neurological diseases. In a case where PV+ nerve cells are produced from induced pluripotent stem cells derived from patients with neurological diseases, it is possible to use the obtained PV+ nerve cells as a model for neurological diseases. Such PV+ nerve cells are useful for elucidating the mechanisms of neurological diseases and the like.

In the production method of the present embodiment, Ascl1 is a transcription factor belonging to the bHLH family that works in the early stage of neurogenesis. Dlx2 is expressed in cells derived from the cerebral basal ganglia primordium and is an important transcription factor in the production of GABAergic nerve cells in the cerebrum. MEF2C is a transcription factor exhibiting a particularly high expression in myocytes and nerve cells. Examples of NCBI accession numbers for human and mouse Ascl1, Dlx2 and MEF2C proteins, and mRNA are shown in Tables 1 and 2 below.

[Table 1]

| Protein | Human | Mouse |
|---------|-------|-------|
| Ascl1 | NP_004307.2 | NP_032579.2 |
| Dlx2 | NP_004396.1 | NP_034184.1 |
| MEF2C | NP_001351262.1 | NP_001334503.1 |

[Table 2]

| mRNA | Human | Mouse |
|---|---|---|
| Ascl1 | NM_004316.4 | NM_008553.5 |
| Dlx2 | NM_004405.4 | NM_010054.2 |
| MEF2C | NM_001364333.2 | NM_001347574.1 |

Each of the factors of the Ascl1, Dlx2, and MEF2C may have mutations as long as it has an activity of inducing differentiation into PV+ nerve cells. In a case where each of the factors that induce differentiation into PV+ nerve cells has a mutation, the factors preferably have 80% or more of the sequence identity, more preferably 90% or more of the sequence identity, and even more preferably 95% or more of the sequence identity to the protein or mRNA identified by the NCBI accession numbers exemplified in Table 1 or Table 2.

Here, the sequence identity of the amino acid sequence is a value that indicates the ratio of the amino acid sequence of the target (target amino acid sequence) that matches an amino acid sequence as a reference (reference amino acid sequence). It is possible to determine the sequence identity of the target amino acid sequence to the reference amino acid sequence, for example, in the following manner. First, the reference amino acid sequence and the target amino acid sequence are aligned. Here, each amino acid sequence may include gaps to maximize the sequence identity. Subsequently, it is possible to calculate the number of matched amino acids in the reference amino acid sequence and the target amino acid sequence and to obtain the sequence identity according to Equation (1).

$$\text{Sequence identity (\%)} = \text{Number of matched amino acids/Total number of amino acids in the target amino acid sequence} \times 100 \ ... \ (1)$$

In the same manner, it is possible to determine the sequence identity of a target base sequence to a reference base sequence, for example, in the following manner. First, the reference base sequence and the target base sequence are aligned. Here, each base sequence may include gaps to maximize the sequence identity. Subsequently, the number of matched bases in the reference base sequence and the target base sequence is calculated and it is possible to obtain the sequence identity according to Equation (2).

$$\text{Sequence identity (\%)} = \text{Number of matched bases/Total number of bases in the target base sequence} \times 100 \ ... \ (2)$$

In the production method of the present embodiment, the factor that induces differentiation into PV+ nerve cells may be a protein or may be a gene (nucleic acids) that encodes the protein. In addition, the gene (nucleic acids) may be mRNA or may be DNA. In a case where the factor described above is DNA, the DNA may be included in an expression vector.

In the production method of the present embodiment, as long as it is possible to induce the expression, Ascl1 gene, Dlx2 gene, and MEF2C gene may be endogenous to the cell or may be introduced from outside in an expressible manner. In a case where the genes are introduced from outside in an expressible manner, the expression induction step includes a gene introduction step in which Ascl1 gene and Dlx2 gene are introduced in an expressible manner. The gene introduction step may further include a gene introduction step to introduce MEF2C gene in an expressible manner. In the gene introduction step, for example, it is possible to construct an expression vector including Ascl1 gene, Dlx2 gene, and MEF2C gene to be introduced into the cell.

In the production method of the present embodiment, it is possible to control the expression of Ascl1 gene, Dlx2 gene, and MEF2C gene by an expression cassette for which it is possible to adjust the expression in response to an external stimulus. Such an expression cassette is a nucleic acid construct that includes at least a promoter capable of inducing the expression of downstream genes in response to an external stimulus and Ascl1 gene, Dlx2 gene, and MEF2C gene for which the expression is controlled by the promoter.

The promoter is not particularly limited as long as the promoter is capable of inducing expression of the downstream genes in response to an external stimulus and examples thereof include promoters capable of inducing expression of the downstream genes by binding of a complex between a tetracycline-based antibiotic and a tetracycline trans-activator in a case where the external stimulus is the presence of a tetracycline-based antibiotic (tetracycline or a tetracycline derivative such as doxycycline). On the other hand, in a case where the external stimulus is the absence of the tetracycline-based antibiotic, examples thereof include promoters capable of inducing expression of the downstream genes by dissociation of the tetracycline repressor. In addition, in a case where the external stimulus is the presence of ecdysteroids (ecdysone, mullisterone A, ponasterone A, or the like), examples thereof include promoters capable of inducing expression of the downstream genes by binding of the ecdysteroid to the ecdysone receptor-retinoid receptor complex. Furthermore, in a case where the external stimulus is the presence of FKCsA, examples thereof include promoters capable of inducing expression of the downstream genes by the binding of FKCsA to a VP16 activator domain complex fused to Gal4 DNA binding domain cyclophilin fused to FKBP12.

The expression cassette may include enhancers, silencers, selection marker genes (for example, drug resistance genes such as neomycin resistance genes), SV40 replication origins, and the like, as necessary. In addition, a person skilled in the art is able to construct an expression cassette capable of inducing the expression of Ascl1 gene, Dlx2 gene, and MEF2C gene at a desired expression level by appropriately selecting and combining enhancers, silencers, selection marker genes, terminators, and the like from examples known in the art, in consideration of the type or the like of the promoter to be utilized.

In this manner, external stimulus includes culturing in the presence or absence of drugs. For example, a tetracycline expression induction system (for example, Takara or the like) is used to control the expression of target genes in the presence of doxycycline. For example, it is possible to produce vectors to be able to express Ascl1 gene, Dlx2 gene, and MEF2C gene under the control of a tetracycline-regulated (Tet-ON) promoter. That is, in the production method of the present embodiment, Ascl1 gene, Dlx2 gene, and MEF2C gene may be tetracycline-regulated (Tet-ON).

Next, the Ascl1 gene, the Dlx2 gene, and the MEF2C gene expression vectors are transfected into cells to produce transgenic cells. On the other hand, a Tet-ON regulatory plasmid expressing a reverse tetracycline-regulated trans-activating factor (rtTA) is produced and the regulatory plasmid is introduced into the cells. When doxycycline is added to a medium, the rtTA binds to the tetracycline response factor to induce downstream gene expression. In addition, when doxycycline is not present in the medium, the expression of the downstream target gene is not induced.

The tetracycline-regulated expression systems may use commercially available products (for example, Knockout™ Tet RNAi System P (Clontech, Inc.)) or may be produced by the method described in Dickins RA. et al.; (Nature Genetics, 39(7): 914-921 (2007)).

In the production method of the present embodiment, at the same time as the expression induction of Ascl1 gene, Dlx2 gene, and MEF2C gene, it is preferable to further induce the expression of at least one selected from the group consisting of microRNA-9/9* (miRNA-9/9*), microRNA-124 (miRNA-124), and BclxL gene and it is more preferable to express microRNA-9/9* (miRNA-9/9*), microRNA-124 (miRNA-124), and BclxL gene all at the same time. MicroRNA-9/9* (miRNA-9/9*), microRNA-124 (miRNA-124), and BclxL gene may be endogenous or may be introduced from outside in an expressible manner. In a case where the genes are introduced from outside in an expressible manner, the expression induction step includes a gene introduction step of introducing at least one selected from the group consisting of microRNA-9/9* (miRNA-9/9*), microRNA-124 (miRNA-124), and BclxL gene in an expressible manner. In a case where the introduction is carried out from outside in an expressible manner, it is possible to carry out the introduction into the cell by constructing an expression cassette in the same manner as for Ascl1 gene, Dlx2 gene, and MEF2C gene. In addition, it is possible to produce vectors to be able to express microRNA-9/9* (miRNA-9/9*), microRNA-124 (miRNA-124), and BclxL gene under the control of a tetracycline-regulated (Tet-ON) promoter. That is, microRNA-9/9* (miRNA-9/9*), microRNA-124 (miRNA-124), and BclxL gene may be tetracycline-regulated (Tet-ON).

The NCBI accession numbers for the nucleic acid sequences of human miRNA-9/9* and human miRNA-124 are NR_029692.1 and NR_029669.1, respectively. In addition, the NCBI accession numbers for the nucleic acid sequences of mouse miRNA-9/9* and mouse miRNA-124 are NR_029818.1 and NR_029814.1, respectively. In addition, the NCBI accession numbers for the protein and mRNA of human BclxL are NP_001309169.1 and NM_001322240.2, respectively. The NCBI accession numbers for the protein and mRNA of mouse BclxL are NP_001341982.1 and NM_001355053.1, respectively.

In the production method of the present embodiment, the method of introducing the expression cassette into a cell is not particularly limited and it is possible to appropriately select and use any known method. For example, it is possible to perform the introduction by a known phenotypic transformation method such as a viral infection method in which the expression cassette is inserted into an appropriate expression vector and a viral vector such as a retroviral vector or an adenoviral vector is used, a lipofection method, a liposome method, an electroporation method, a calcium phosphate method, a DEAE dextran method, or a microinjection method.

Expression vectors are not particularly limited as long as it is possible to express these genes in cells and the expression vectors may be plasmid vectors, viral vectors, or transposon vectors. Viral vectors are easy to use due to having a high gene introduction efficiency. Examples of viral vectors include retroviral vectors, lentiviral vectors, adeno-associated viral vectors, adenoviral vectors, and the like. In a case where transposon vectors or lentiviral vectors are used, it is possible to insert a plurality of copies of Ascl1 gene, Dlx2 gene, and MEF2C gene, microRNA-9/9* (miRNA-9/9*), microRNA-124 (miRNA-124), and BclxL gene into the genome. Due to this, it is possible to maintain the cells for neural induction in a tetracycline-regulated (Tet-ON) state. Furthermore, it is possible to eliminate cells where genomic insertion of genes or the like did not take place by the external stimulus described above and maintain only cells which are able to be induced to differentiate into PV+ nerve cells. In addition, it is possible to completely remove the transposon vector from the cells as necessary. Examples of such transposon vectors include Piggybac transposon vectors and the like.

In addition, in relation to the external stimulus described above, it is possible for a person skilled in the art to appropriately prepare the amount to be added to the medium described below, in consideration of the type of the promoter to be used and the like. For example, in a case where the external stimulus is the presence of doxycycline, a suitable concentration of doxycycline to be added is 0.1 to 10 μg/ml and more preferably 1 to 2 μg/ml.

For the expression induction step, in which the expression of Ascl1 gene, Dlx2 gene, and MEF2C gene is induced in the cell, examples of the culture medium to which the external stimulus is added include a medium for nerve cells, for example, Neurobasal Plus Medium (manufactured by Thermo Fisher Scientific) and the like. The culture medium may include additives normally added to the culture. Examples of the additives include γ-selectase inhibitors, dibutyryl cAMP (dbcAMP), ROCK inhibitors such as Y27632, and the like. Examples of γ-selectase inhibitors include DAPT (γ-secretase inhibitor IX), Compound E, γ-secretase inhibitor XI, γ-secretase inhibitor III, and the like. However, it is not necessary to add serum in the expression induction step and the subsequent differentiation step and, in one aspect of the production method of the present embodiment, the induction of differentiation into PV+ nerve cells is performed in the absence of a serum.

In the production method of the present embodiment, the cells may be dissociated into single cells and re-seeded before inducing expression of the Ascl1 gene, the Dlx2 gene, and the MEF2C gene. Here, to be dissociated into single cells means to dissociate the cells attached to the culture container one by one. It is possible to perform the dissociation into single cells by performing an enzymatic process with Accutase, Trypsin, Collagenase, TrypLE Select [TrypLE (registered trademark) Select], or the like, which are normally used for cell dissociation, and pipetting, or the like.

For cells including Ascl1 gene, Dlx2 gene, and MEF2C gene, or cells into which Ascl1 gene, Dlx2 gene, and MEF2C gene were introduced in an expressible manner, the expression induction step is performed for 1 to 8 days and preferably 1 to 5 days. In the case of an external stimulus such as doxycycline, the external stimulus is present for 1 to 8 days and preferably 1 to 5 days. The cells are subsequently differentiated into PV+ nerve cells as a result of the differentiation step in which culturing is carried out after the expression induction step is completed. After completion of the expression induction step, the cells are cultured for 10 days or more and preferably 20 days or more. Examples of the medium used in the differentiation step include a medium for nerve cells, for example, Neurobasal Plus Medium (manufactured by Thermo Fisher Scientific) and the like. In the differentiation step, additives normally added to the culture may be included. Examples of the additives include dibutyryl cAMP (dbcAMP), BDNF, GDNF, ascorbic acid, and the like.

By the differentiation step after the completion of the expression induction step, the cells including the gene are differentiated into PV+ nerve cells. It is possible to confirm the differentiation into PV+ nerve cells, for example, by the expression of PV in the nerve cells during a certain period of time (for example, 40 days) after the completion of the expression induction step. In addition, confirmation is possible by the expression of the nerve cell marker TUBB3 and genes such as GRIA4, SYT1, and NRXN2a, which are thought to be increased in PV+ nerve cells. For example, in a case where, in a population of cells, Ascl1 gene, Dlx2 gene, and MEF2C gene are introduced, microRNA-9/9* (miRNA-9/9*), microRNA-124 (miRNA-124), and BclxL gene are further introduced, and an external stimulus with doxycycline is applied, it is possible to confirm that at least 70%, and at least 85% of the population, are differentiated into PV+ nerve cells within 5 to 20 days, preferably 10 to 40 days after the completion of the external stimulation.

[Expression-induced cells]

In one embodiment, the present invention provides cells in which Ascl1 gene, Dlx2 gene, and MEF2C gene are introduced in an expressible manner. It is possible to differentiate the cells of the present embodiment into PV+ nerve cells due to the introduction of Ascl1 gene, Dlx2 gene, and MEF2C gene in an expressible manner. In the cells of the present embodiment, at least one selected from the group consisting of microRNA-9/9* (miRNA-9/9*), microRNA-124 (miRNA-124), and BclxL gene, and preferably microRNA-9/9* (miRNA-9/9*), microRNA-124 (miRNA-124), and BclxL gene may be further introduced in an expressible manner. By further introducing MEF2C gene in addition to Ascl1 gene and Dlx2 gene, progenitor cells with high differentiation efficiency are created and, in addition, by introducing at least one selected from the group consisting of microRNA-9/9* (miRNA-9/9*), microRNA-124 (miRNA-124), and BclxL gene, preferably microRNA-9/9* (miRNA-9/9*), microRNA-124 (miRNA-124), and BclxL gene in an expressible manner, it is possible to obtain PV+ nerve cells in a short period of time with high efficiency.

In the expression-induced cells of the present embodiment, Ascl1 gene, Dlx2 gene, and MEF2C gene may be tetracycline-regulated (Tet-ON). In addition, in a case where, in the cells of the present embodiment, at least one selected from the group consisting of microRNA-9/9* (miRNA-9/9*), microRNA-124 (miRNA-124), and BclxL gene and preferably microRNA-9/9* (miRNA-9/9*), microRNA-124 (miRNA-124), and BclxL gene are further introduced in an expressible manner, microRNA-9/9* (miRNA-9/9*), microRNA-124 (miRNA-124), and BclxL gene may be tetracycline-regulated (Tet-ON).

It is possible to produce the cells to be induced to differentiate into PV+ nerve cells in the present embodiment by the method described in the expression induction step of the production method described above.

In the cells of the present embodiment, examples of cells into which Ascl1 gene, Dlx2 gene, and the MEF2C gene are introduced in an expressible manner are not particularly limited as long as the cells are able to express Ascl1 gene, Dlx2 gene, and MEF2C gene and able to be differentiated into PV+ nerve cells and examples thereof include fibroblasts, mesenchymal stem cells, pluripotent stem cells, and the like, but fibroblasts and pluripotent stem cells are preferable.

[Differentiation inducers]

In one embodiment, the present invention provides a differentiation inducer for inducing cells to differentiate into PV+ nerve cells, the differentiation inducer containing Ascl1 gene, Dlx2 gene, and MEF2C gene, or gene products thereof, as active ingredients. The differentiation inducer of the present embodiment is able to impart the ability to differentiate into PV+ nerve cells to undifferentiated cells that do not have the ability to differentiate into PV+ nerve cells. The differentiation inducer of the present embodiment may further contain at least one selected from the group consisting of microRNA-9/9* (miRNA-9/9*), microRNA-124 (miRNA-124), and BclxL gene and preferably contains microRNA-9/9* (miRNA-9/9*), microRNA-124 (miRNA-124), and BclxL gene, as active ingredients. By containing at least one selected from the group consisting of microRNA-9/9* (miRNA-9/9*), microRNA-124 (miRNA-124), and BclxL gene in addition to Ascl1 gene, Dlx2 gene, and MEF2C gene and preferably containing microRNA-9/9* (miRNA-9/9*), microRNA-124 (miRNA-124), and BclxL gene in addition to Ascl1 gene, Dlx2 gene, and MEF2C gene, as active ingredients, it is possible to obtain PV+ nerve cells in a shorter period of time and with higher efficiency. Accordingly, the differentiation inducer of the present invention is a differentiation inducer further including at least one selected from the gene group consisting of microRNA-9/9* (miRNA-9/9*), microRNA-124 (miRNA-124), and BclxL gene in addition to the Ascl1 gene, the Dlx2 gene, and the MEF2C gene and preferably further including microRNA-9/9* (miRNA-9/9*), microRNA-124 (miRNA-124), and BclxL gene in addition to the Ascl1 gene, the Dlx2 gene, and the MEF2C gene.

In the differentiation inducer of the present embodiment, Ascl1 gene, Dlx2 gene, and MEF2C gene may be tetracycline-regulated (Tet-ON). In addition, in a case where the differentiation inducer of the present embodiment further contains at least one selected from the group consisting of microRNA-9/9* (miRNA-9/9*), microRNA-124 (miRNA-124), and BclxL gene, preferably microRNA-9/9* (miRNA-9/9*), microRNA-124 (miRNA-124), and BclxL gene, as an active ingredient, microRNA-9/9* (miRNA-9/9*), microRNA-124 (miRNA-124), and BclxL gene may be tetracycline-regulated (Tet-ON).

In the differentiation inducer of the present embodiment, the active ingredients which induce differentiation into PV+ nerve cells may be Ascl1 gene, Dlx2 gene, and MEF2C gene, or may be gene products thereof. The gene products may be in the form of the proteins produced by the genes or in the form of fusion gene products of the proteins with other proteins, peptides or the like. For example, it is also possible to use a fusion protein with green fluorescent protein (GFP) or a fusion gene product with a peptide such as a histidine tag. Methods for preparing such fusion gene products are well known and it is possible for a person skilled in the art to easily design and prepare suitable fusion gene products according to the purpose.

The genes may be included in an expression vector. That is, the differentiation inducer of the present embodiment may be an expression vector into which the gene which induces differentiation into PV+ nerve cells is introduced in an expressible manner. An expression vector or the like listed in the expression induction step of the production method described above may be used as the expression vector.

By introducing the differentiation inducer of the present embodiment into a cell, it is possible to induce the cell to differentiate into PV+ nerve cells. In a case where the differentiation inducer of the present embodiment is a protein, RNA, or the like, examples of methods of introducing the differentiation inducer into the cell include contacting cells, microinjection, methods using virus-like particles, a lipofection method, combinations of these methods, and the like.

In addition, in a case where the differentiation inducer of the present embodiment is a vector or the like, examples of methods of introducing the differentiation inducer into cells include contacting cells, a lipofection method, an electroporation method, a microinjection method, a DEAE-dextran method, a calcium phosphate method, combinations thereof, and the like. In addition, in a case where the vector is a viral vector, introduction into a cell by infecting the cell is also possible.

The introduction of the differentiation inducer of the present embodiment into the cell may be performed in vitro or may be performed in vivo. In addition, one type of differentiation inducer may be used alone, or two or more types may be mixed and used.

The cells that the differentiation inducer of the present embodiment induces to differentiate are not particularly limited as long as the cells are able to be induced into PV+ nerve cells and examples thereof include fibroblasts, mesenchymal stem cells, pluripotent stem cells, and the like, but fibroblasts and pluripotent stem cells are preferable.

In addition, examples of cell species that the differentiation inducer of the present embodiment induces to differentiate include human, mouse, rat, rabbit, dog, monkey, pig, goat, sheep, and the like.

**EXAMPLES**

Next, a more detailed description will be given of the present invention using Examples, but the present invention is not limited to the following Examples.

[Experimental Example 1]

(Maintaining and culturing iPS cells)

Culturing was carried out according to the known cell culture method published by the Kyoto University iPS Research Institute, except that iPS cells (1210B2 line) were set in a 6-well plate at an iPS cell seeding density of $1.5 \times 10^4$ cells/well at the time of cell transfer, pre-plate coating was not performed at the time of cell transfer, StemFit medium for undifferentiated cells (AK02N, manufactured by Ajinomoto Co., Inc.) including 1.5 mL of 10 $\mu$g/ml of ROCK inhibitor Y27632 (manufactured by Fuji Film Wako Pure Chemical Corporation) and 1.5 $\mu$g/ml iMatrix-511 (manufactured by Nippi, Inc.) was poured into a 6-well plate immediately before cell seeding at 1.5 ml/well, and direct cell seeding was performed.

[Experimental Example 2]

(Introduction of Ascl1 gene and Dlx2 gene into iPS cells)

The scheme of introduction of Ascl1 gene and Dlx2 gene into iPS cells is shown in Fig. 1A. Specifically, the introduction of Ascl1 gene and Dlx2 gene into the iPS cells was performed as follows.

(1) Preparation of medium of plate for seeding cells after gene introduction operation and coating preparation

In a 6-well plate, 20 $\mu$g/ml of Y27632 (manufactured by Fuji Film Wako Pure Chemical Corporation) and 2.5 $\mu$g/ml of iMatrix-511 (manufactured by Nippi, Inc.) were added to StemFit (AK02N, manufactured by Ajinomoto Co., Inc.) and the result was placed in six well portions (one plate) at 2 ml/well and incubation was performed at 37°C and 5% $CO_2$.

(2) Single cell of iPS cells

iPS cells seeded to be approximately $1.5 \times 10^4$ cells/well in a 6-well plate were cultured for approximately one week in StemFit (AK02N, manufactured by Ajinomoto Co., Inc.), the medium was removed with an aspirator, and then the cells were washed using PBS (-) at 1 ml/well. Next, after removing the PBS (-), $0.5 \times$ TrypLE (registered trademark) Select [a mixture of 0. 5 M EDTA (pH 8.0) in an amount corresponding to an amount of 1/2000 of the total amount in a mixture of TrypLE Select (manufactured by Thermo Fisher Scientific): PBS (-)=1:1] was added at 0.5 ml/well, incubation was carried out at 37°C and 5% $CO_2$ for approximately 5 minutes, it was confirmed that inter-cell adhesion decreased and the outline of each cell was visible to some extent, and then the cells were washed at 1 ml/well using PBS (-).

Next, using 1 ml of StemFit (AK02N, manufactured by Ajinomoto Co., Inc.) including 10 $\mu$g/ml of Y27632 (manufactured by Fuji Film Wako Pure Chemical Corporation), cells were rapidly detached and moved to a 15 ml or 50 ml conical tube and thoroughly stirred, then, 10 $\mu$l of a cell suspension and trypan blue staining solution were mixed therein and added to a hemocytometer, the number of viable cells was calculated, and the viable cell density of the suspension was calculated. The cell suspension was left to stand at 4°C or on ice and a portion of the cells was seeded in a 6-well plate at $1.5 \times 10^4$ cells for use in transfer to maintain the culture.

(3) Introduction into iPS cells of vector into which Ascl1 gene and Dlx2 gene were introduced

A gene introduction reagent Gene Juice (#70967, manufactured by Merck Millipore) for lipofection method was returned to room temperature and stirred well using a vortex mixer or the like and then 4.5 $\mu$l thereof was added to 100 $\mu$l of Opti-MEM (#31985088, manufactured by Thermo Fisher Scientific) in a 1.5-mL tube, stirred well, and left to stand at room temperature for 5 minutes to prepare a lipofection reagent cocktail.

0.4 μg of a transposase expression vector (pCMV-HyPBase-PGK-Puro) shown in the upper row of Fig. 1C and a PiggyBac vector for rtTA expression (pG-PB-CAG-rtTA3G-IH), a PiggyBac vector for Tet-inducible hAscl1 gene expression (PB-P(tetO)-hAscl1-pAPGK-PuroTK-pA), and a PiggyBac vector for Tet-inducible hDlx2 gene expression (PB-p(tetO)-hDlx2-pA-floxPGKneo-pA) shown in the middle row of Fig. 1C were added to the lipofection reagent cocktail prepared as described above, stirred well, and left to stand at room temperature for 15 minutes to prepare a vector lipofection reagent.

During the 15 minutes of being left to stand, the equivalent of $3 \times 10^5$ cells of the cell suspension made into single cells in (2) was moved to a 1.5-ml tube, centrifugation was carried out at 200G $\times$ 5 minutes, and after 15 minutes passed, the supernatant after centrifugation was removed, and the vector lipofection reagent cocktail prepared as described above was added to pellets, pipetted, and left to stand at room temperature for 5 minutes.

The obtained cells were seeded evenly in each well at 15 to 20 μl/well in the cell culture plate previously incubated in (1) and incubated at 37°C and 5% $CO_2$ for approximately three hours. Thereafter, StemFit (AK02N, manufactured by Ajinomoto Co., Inc.) including 20 μg/ml of Y27632 (manufactured by Fuji Film Wako Pure Chemical Corporation) was heated at 37°C and the entire medium was changed for all wells at 2 ml/well.

(4) Selection of iPS cells able to be induced into PV+ nerve cells by antibiotics

One day after the gene introduction in (3), the entire medium was changed (1.5 ml/well) with StemFit (AK02N, manufactured by Ajinomoto Co., Inc.) to which 50 μg/ml of hygromycin and 100 μg/ml of G418 were added and culturing was carried out for two days.

After two days of culturing, the entire medium was changed (1.5 ml/well) with StemFit (AK02N, manufactured by Ajinomoto Co., Inc.) to which 100 μg/ml of hygromycin (Hygromycin B Solution, #09287-84, manufactured by Nacalai Tesque Inc.), 100 μg/ml of G418 (G418 Disulfate, #08973-14, manufactured by Nacalai Tesque Inc.), and 5 μg/ml of puromycin (#ant-pr-1, manufactured by InvivoGen, #14861-71, manufactured by Nacalai Tesque Inc.) were added and culturing was carried out for two days.

After two days of culturing, the entire medium was changed (1.5 ml/well) with StemFit (AK02N, manufactured by Ajinomoto Co., Inc.) to which 200 μg/ml of hygromycin, 100 μg/ml of G418, and 10 μg/ml of puromycin were added and culturing was carried out for two days.

After two days of culturing, the entire medium was changed (1.5 ml/well) with StemFit (AK02N, manufactured by Ajinomoto Co., Inc.), cultured for one day without adding antibiotics, and the surviving iPS cells were transferred or cryopreserved as necessary.

[Experimental Example 3]

(Introduction of MEF2C gene, miRNA-9/9*, miRNA-124, and BclxL gene into iPS cells)

The scheme of introduction of MEF2C gene, miRNA-9/9*, miRNA-124, and BclxL gene into the iPS cells is shown in Fig. 1B. Specifically, MEF2C gene, miRNA-9/9*, miRNA-124, and BclxL gene were introduced into the iPS cells as follows.

(1) Purification of lentiviruses into which MEF2C gene, miRNA-9/9*, miRNA-124, and BclxL gene were introduced

In accordance with a normal method, as shown in the lower part of Fig. 1C, lentiviruses into which the MEF2C gene, miRNA-9/9*, miRNA-124, and BclxL gene were introduced were purified. Specifically, lentiviruses into which MEF2C gene, miRNA-9/9*, miRNA-124, and BclxL gene were introduced were purified as follows.

100 mm dishes for cell/tissue culture coated using a solution, in which a 0.01% Poly-L-Lysine solution (0.01%, #P4832, manufactured by Sigma) was mixed in PBS (-) at 1:100, were washed with PBS (-), and then HEK293T cells cultured semi-confluently in a DMEM medium (#D5796, manufactured by Sigma) including 10% FBS and 2 mM L-glutamine were well mixed with 500 μl of HBSS, 3 μg of packaging vector (pCAG-HIVgp), 3 μg of envelope generation vector/Rev expression vector (VSV-G, REV expression vector, pCMV-VSV-G-RSV-Rev), 6 μg of SIN vector (Tet-sensitive miRNA-9/9*, miRNA-124, BclxL gene expression vector, CSIV-124-9-BClxL-TRE-EF-BsdT), 6 μg of SIN vector (Tet-sensitive MEF2C gene expression vector, pLV-TRE3G-HA-hMEF2C-mPGK-Zeo), and 24 μl of polyethyleneimine (molecular weight: 25,000, #23966, manufactured by Polysciences Inc.) [1 mg/ml in Milli-Q (registered trademark) water] with respect to one dish and left to stand at room temperature for 15 minutes, and then dropped evenly into the dish.

After incubation at 37°C and 5% $CO_2$ for 12 hours, the medium was changed with DMEM (#D5796, manufactured by Sigma) including 10 μM of forskolin and incubated again at 37°C and 5% $CO_2$. After 48 hours, the cell culture supernatant was removed and passed through a 0.45 μm syringe filter to remove floating cells and the like, then, micro pellets obtained after centrifugation at 50,000 $\times$ G for two hours using an ultracentrifuge were collected in PBS (-) and then the virus titer was estimated.

(2) Infection of iPS cells with lentiviral vector for miRNA-9/9*, miRNA-124, and BclxL gene introduction, and lentiviral vector for MEF2C gene introduction

StemFit (AK02N, manufactured by Ajinomoto Co., Inc.) including 10 $\mu$g/ml of Y27632 (manufactured by Fuji Film Wako Pure Chemical Corporation) and 1.5 of $\mu$g/ml iMatrix-511 (manufactured by Nippi, Inc.) was placed in a 6-well plate, the iPS cells prepared in Experimental Example 2 were seeded at $1.5 \times 10^4$ cells/well, and culturing was carried out for one day.

Next, the entire medium was changed with StemFit (AK02N, manufactured by Ajinomoto Co., Inc.) to which Y27632 and iMatrix-511 were not added at 1.5 ml/well and then infection was carried out by adding the virus suspension prepared in (1) (each MOI = 2.0) and culturing was carried out for two days.

(3) Selection of lentivirus-infected iPS cells by antibiotics

Two days after the addition of the virus suspension of (2), the entire medium was changed with StemFit (AK02N, manufactured by Ajinomoto Co., Inc.) at 1.5 ml/well and 20 $\mu$g/ml of Blastcidin or 200 $\mu$g/ml of Zeocin was added thereto and culturing was carried out for two days.

After culturing for two days, the entire medium was changed with StemFit (AK02N, manufactured by Ajinomoto Co., Inc.) at 1.5 ml/well and 20 $\mu$g/ml of Blasticidin (Blasticidin S, hydrochloride, #KK-400, manufactured by Funakoshi Co., Ltd.) or 200$\mu$ g/ml of Zeocin (Zeocin solution, #ant-zn-1, manufactured by InvivoGen, #61483-26, manufactured by Nacalai Tesque Inc.) described above was added in the same manner as described above and culturing was carried out for one day.

Thereafter, the entire medium was changed with StemFit (AK02N, manufactured by Ajinomoto Co., Inc.) not including the antibiotics described above and the surviving iPS cells were transferred or cryopreserved as necessary.

[Experimental Example 4]

(Differentiation into PV+ nerve cells of iPS cells for which gene introduction was completed)

(1) Pre-coating of cell culture plate

A Poly-L-Lysine solution (0.01%, #P4832, manufactured by Sigma) was diluted 100-fold in PBS (-) and poured onto a plate to be coated for six hours or more. As plates for cell collection for RNA analysis or the like, 6-well plates were used and coated at 1.5 ml/well. As plates for immunostaining and imaging, glass-bottomed 96-well plates were used and coated at 100 $\mu$l/well. During the coating, the plates were placed in a $CO_2$ incubator or the like for cell culturing and kept at a constant humidity to prevent drying out.

One hour before seeding iPS cells for neural differentiation, the Poly-L-Lysine solution was removed by suction and washing was carried out three times with PBS (-). Washing was performed at approximately 1 ml/well each for the 6-well plates and 100 $\mu$l/well each for the 96-well plates and the residual liquid was sufficiently removed by suction after three washes.

Next, using a 50 ml conical tube or the like, a solution in which Growth Factor Reduced Matrigel (#354230, manufactured by Corning Inc.) was diluted 50-fold in PBS (-) was created and the 6-well plates were coated at 1.5 ml/well each and the 96-well plates were coated at 100 $\mu$l/well each therewith.

In the Matrigel coating described above, iPS cells for neural differentiation were made into single cells by the same method as in (2) of Experimental Example 2 and the cell density of the cell suspension was calculated.

(2) Differentiation into PV+ nerve cells

As mediums for differentiation, mediums mixed as described below were prepared.

- Neurobasal Plus Medium (#A3582901, manufactured by Thermo Fisher Scientific, mixed with the following as basic mediums)
- B27 Plus Supplement (50×, #A3582801, manufactured by Thermo Fisher Scientific)

(1:50)

- Glutamax (manufactured by Thermo Fisher Scientific) (1:100)
- dbcAMP (N6,2'-O-Dibutyryladenosine-3',5'-cyclic Monophosphate Sodium Salt, #11540-61, manufactured by Nacalai Tesque Inc.) (100 $\mu$M)
- DAPT [N-[N-(3,5-Difluorophenacetyl-L-alanyl)]-(S)-phenylglycine t-butyl ester, #D5942-5MG, manufactured by Sigma) (10 $\mu$M)
- Doxycycline (#D4116, manufactured by Tokyo Chemical Industry Co., Ltd.) (2.0 $\mu$g/ml)
- Y27632 (#030-24026, manufactured by Fuji Film Wako Pure Chemical Corporation)

(20 $\mu$g/ml)

Immediately before pouring into the plate, 1.0 $\mu$g/ml of iMatrix-511 (manufactured by Nippi, Inc.) was added to the mediums for differentiation described above and the mediums were poured at 2.0 ml/well for the 6-well plates and at 100 $\mu$l/well for the 96-well plates. Next, the iPS cell suspension prepared in Experimental Example 3 was seeded evenly at $6.0 \times 10^5$ cells/well for the 6-well plates and $3.0 \times 10^4$ cells/well for the 96-well plates.

(3) Culture of PV+ nerve cells

The mediums of the iPS cells differentiated in (2) were completely changed with the following mediums on the fifth day after the start of culturing. The mediums were poured at 2.0 ml/well for the 6-well plates and at 100 $\mu$l/well each for the 96-well plates.

- Neurobasal Plus Medium (#A3582901, manufactured by Thermo Fisher Scientific, mixed with the following as basic mediums)
- B27 Plus Supplement (50$\times$, #A3582801, manufactured by Thermo Fisher Scientific)

(1:50)

- Glutamax (manufactured by Thermo Fisher Scientific) (1:100)
- dbcAMP (N6,2'-O-Dibutyryladenosine-3',5'-cyclic Monophosphate Sodium Salt, #11540-61, manufactured by Nacalai Tesque Inc.) (100 $\mu$M)
- BDNF (Recombinant human BDNF protein, #B-250, manufactured by Alomone Labs) (10 ng/ml)
- GDNF (Recombinant human GDNF protein, #G-240, manufactured by Allomone Labs) (10 ng/ml)
- L-Ascorbic Acid (#016-04805, manufactured by Fuji Film Wako Pure Chemical Corporation) (200 $\mu$M)

Culturing was performed by replacing half of the medium of the above composition every five days and, after the tenth day of culturing, RNA purification by cell collection, immunostaining by cell fixation, and the like were performed.

[Experimental Example 5]

(Preparation of parvalbumin gene (PVALB) reporter cell line)

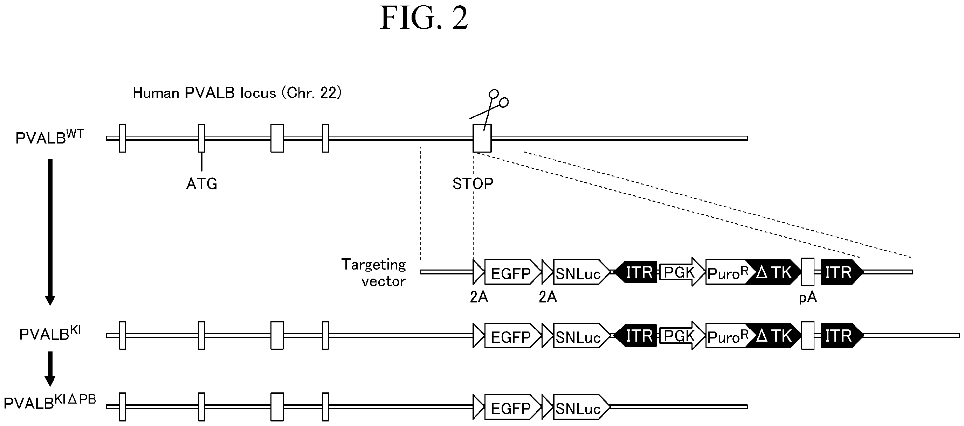

Fig. 2 shows the scheme for the preparation of a parvalbumin gene (PVALB) reporter cell line. Specifically, the parvalbumin gene (PVALB) reporter cell line was prepared as follows.
Using CRISPR/Cas9, EGFP, secreted luciferase (SNLuc), and a puromycin resistance gene driven by a constant expression promoter interposed between PiggyBac ITRs were inserted into the termination codon site of a human parvalbumin gene (PVALB). After gene introduction, homotypic PVALB knock-in cells were acquired by drug selection, then sequences inside the ITR were removed by transposase, and a reporter cell line of EGFP and SNLuc expressed by 2A peptide downstream of the PVALB gene was acquired.

[Experimental Example 6]

(Immunostaining of cells after 20 days following expression induction (5 days of expression induction and 15 days of differentiation))

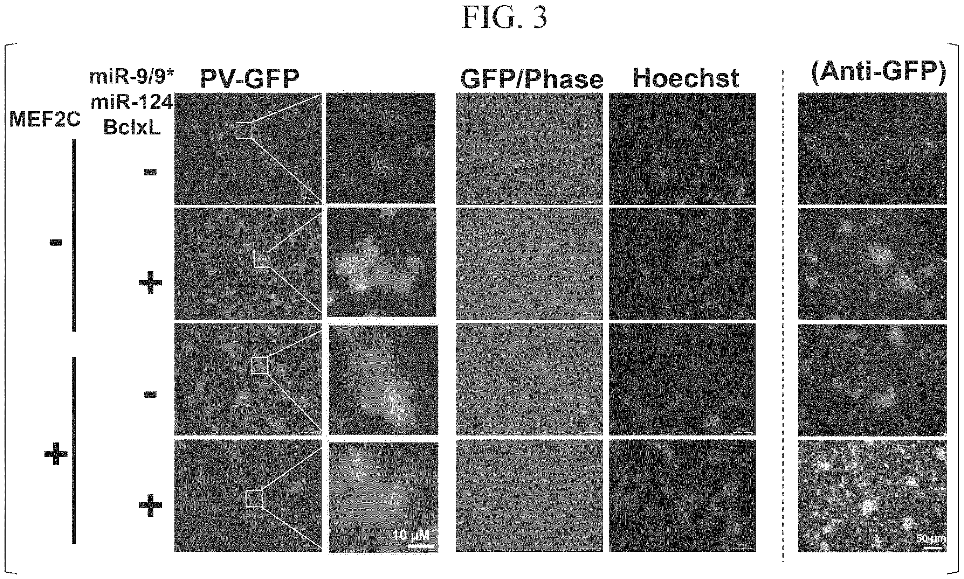

For cells with transient expression of Ascl1 gene, Dlx2 gene, miRNA-9/9*, miRNA-124, and BclxL gene, MEF2C gene transient expression induction and uninduction were performed. After 20 days following the expression induction (5 days of expression induction and 15 days of differentiation), cells were fixed and immunostained with Anti-Tubb3 (nerve cell marker) antibodies, Anti-Parvalbumin (PV) antibodies, and Anti-GFP antibodies. In addition, Hoechst (Ho) staining was also performed as cell nucleus staining. The results are shown in Fig. 3 and Fig. 4.

As shown in Fig. 3 and Fig. 4, it was confirmed that there were more PV+ nerve cells in the MEF2C-induced group. In addition, as shown in Fig. 3 and Fig. 4, it was confirmed that PV+ nerve cells were induced to differentiate in the MEF2C-induced group in the miRNA-9/9*, miRNA-124, and BclxL gene non-induced groups.

[Experimental Example 7]

(Analysis of mRNA expression level of parvalbumin by quantitative PCR method)

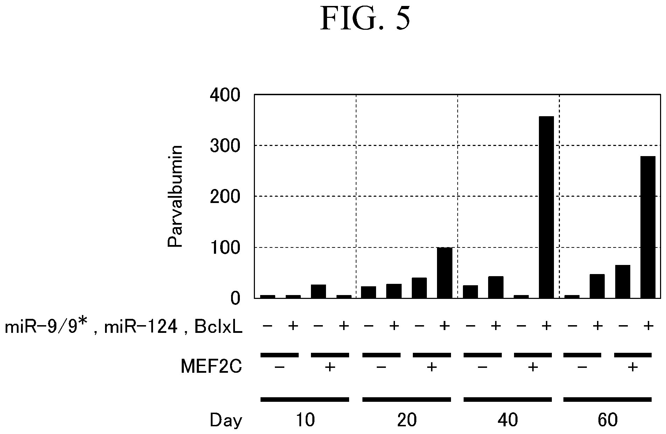

Under inhibitory nerve cell induction by Ascl1 gene and Dlx2 gene, for each combination of miRNA-9/9*, miRNA-124, and BclxL gene introduction, and MEF2C gene introduction, cells were collected over time at 10 days, 20 days, 40 days, and 60 days after the expression induction and quantitative PCR for parvalbumin mRNA was performed. The results are shown in Fig. 5.

As shown in Fig. 5, at 20 days (day 20), 40 days (day 40), and 60 days (day 60) after expression induction, co-expression of both the miRNA-9/9*, miRNA-124, and BclxL gene and the MEF2C gene synergistically increased the level of parvalbumin gene expression compared to the expression of one alone.

Industrial Applicability

According to the present invention, it is possible to provide a production method for parvalbumin-positive nerve cells with high efficiency in a short period of time with few differentiation induction steps, cells able to be induced into the PV+ nerve cells, and a differentiation inducer for inducing differentiation into the PV+ nerve cells.

**Claims**

1. A production method for parvalbumin-positive nerve cells, comprising:

   an expression induction step of inducing expression of Ascl1 gene, Dlx2 gene, and MEF2C gene in a cell; and
   a differentiation step of culturing the cells after the expression induction to differentiate the cells into parvalbumin-positive nerve cells.

2. The production method for parvalbumin-positive nerve cells according to Claim 1, wherein the expression induction step comprises a gene introduction step of introducing Ascl1 gene and Dlx2 gene into the cell in an expressible manner.

3. The production method for parvalbumin-positive nerve cells according to Claim 2, wherein the expression induction step further comprises a gene introduction step of introducing MEF2C gene in an expressible manner.

4. The production method according to any one of Claims 1 to 3, wherein the Ascl1 gene, the Dlx2 gene, and the MEF2C gene are tetracycline-regulated (Tet-ON).

5. The production method according to any one of Claims 1 to 4, wherein the expression induction step comprises a step of simultaneously inducing expression of at least one selected from the group consisting of microRNA-9/9* (miRNA-9/9*), microRNA-124 (miRNA-124), and BclxL gene.

6. The production method according to Claim 5, wherein the expression induction step includes a gene introduction step of introducing at least one selected from the group consisting of microRNA-9/9* (miRNA-9/9*), microRNA-124 (miRNA-124), and BclxL gene in an expressible manner.

7. The production method according to Claim 5 or 6, wherein the microRNA-9/9* (miRNA-9/9*), the microRNA-124 (miRNA-124), and the BclxL gene are tetracycline-regulated (Tet-ON).

8. The production method according to any one of Claims 1 to 7, wherein the cells are fibroblasts or pluripotent stem cells.

9. The production method according to any one of Claims 1 to 8, wherein the expression induction step is performed for 1 to 5 days.

10. The production method according to any one of Claims 1 to 9, wherein the differentiation step is performed for 10 days or more.

11. A cell wherein Ascl1 gene, Dlx2 gene, and MEF2C gene are introduced in an expressible manner.

12. The cell according to Claim 11, wherein the Ascl1 gene, the Dlx2 gene, and the MEF2C gene are tetracycline-regulated (Tet-ON).

13. The cell according to Claim 11 or 12, wherein at least one selected from the group consisting of microRNA-9/9* (miRNA-9/9*), microRNA-124 (miRNA-124), and BclxL gene is further introduced in an expressible manner.

14. The cell according to Claim 13, wherein the microRNA-9/9* (miRNA-9/9*), the microRNA-124 (miRNA-124), and the BclxL gene are tetracycline-regulated (Tet-ON).

15. The cell according to any one of Claims 11 to 14, wherein the cell is a fibroblast or a pluripotent stem cell.

16. A differentiation inducer for inducing differentiation of a cell into a parvalbumin-positive nerve cell, the differentiation inducer comprising: Ascl1 gene, Dlx2 gene, and MEF2C gene, or a gene product thereof, as an active ingredient.

17. The differentiation inducer according to Claim 16, wherein the Ascl1 gene, the Dlx2 gene, and the MEF2C gene are tetracycline-regulated (Tet-ON).

18. The differentiation inducer according to Claim 16 or 17, further comprising: at least one selected from the group consisting of microRNA-9/9* (miRNA-9/9*), microRNA-124 (miRNA-124), and BclxL gene, or gene products thereof, as an active ingredient.

19. The differentiation inducer according to Claim 18, wherein the microRNA-9/9* (miRNA-9/9*), the microRNA-124 (miRNA-124), and the BclxL gene are tetracycline-regulated (Tet-ON).

20. The differentiation inducer according to any one of Claims 16 to 19, wherein the cells are fibroblasts or pluripotent stem cells.

FIG. 1A

FIG. 1B

FIG. 1C

# FIG. 2

Human PVALB locus (Chr. 22)

PVALB^WT

ATG — STOP

Targeting vector: 2A — EGFP — 2A — SNLuc — ITR — PGK — PuroR — ΔTK — pA — ITR

PVALB^KI: EGFP — SNLuc — ITR — PGK — PuroR — ΔTK — ITR

PVALB^KIΔPB: EGFP — SNLuc

FIG. 3

FIG. 4

# FIG. 5

EP 4 023 748 A1

## INTERNATIONAL SEARCH REPORT

| International application No. |
| --- |
| PCT/JP2020/032606 |

A. CLASSIFICATION OF SUBJECT MATTER
C12N 5/0793(2010.01)i; C12N 5/10(2006.01)i; C12N 15/113(2010.01)i
FI: C12N5/0793; C12N5/10; C12N15/113 Z

According to International Patent Classification (IPC) or to both national classification and IPC

B. FIELDS SEARCHED

Minimum documentation searched (classification system followed by classification symbols)
C12N5/0793; C12N5/10; C12N15/113

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
| --- | --- | --- |
| X | US 2013/0022583 A1 (WERNIG, Marius) 24 January 2013 (2013-01-24) paragraphs [0152], [0154], [0157], [0166], [0184], [0189], [0202] | 1-4, 8, 10-12, 15-17, 20 |
| Y | paragraphs [0152], [0154], [0157], [0166], [0184], [0189], [0202], [0261] | 1-20 |
| Y | JP 2018-513686 A (AGENCY FOR SCIENCE, TECHNOLOGY AND RESEARCH) 31 May 2018 (2018-05-31) claim 62, paragraphs [0053], [0062], [0077], example 1 | 1-20 |
| Y | KAMATH, S. P. et al., "Myocyte Enhancer Factor 2c Regulates Dendritic Complexity and Connectivity of Cerebellar Purkinje Cells", Mol. Neurobiol., 2019, vol. 56, pp. 4102-4119, Published online: 01 October 2018 fig. 2, page 4114, left column, lines 14-16 | 1-20 |

☐ Further documents are listed in the continuation of Box C.    ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 23 October 2020 (23.10.2020) | 02 November 2020 (02.11.2020) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

Information on patent family members

International application No.

PCT/JP2020/032606

| Patent Documents referred in the Report | Publication Date | Patent Family | Publication Date |
|---|---|---|---|
| US 2013/0022583 A1 | 24 Jan. 2013 | WO 2011/091048 A1 | |
| JP 2018-513686 A | 31 May 2018 | US 2018/0072988 A1 claim 81, paragraphs [0073], [0082], [0097], example 1 | |

Form PCT/ISA/210 (patent family annex) (January 2015)

**EP 4 023 748 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019157194 A

**Non-patent literature cited in the description**

- **LEWIS DA et al.** *Trends Neurosci.,* January 2012, vol. 35 (1), 57-67
- **RODRIGUEZ RA et al.** *Front Mol Neurosci.,* 24 April 2018, vol. 11, 132
- **YANG N. et al.** *Nat Methods,* June 2017, vol. 14 (6), 621-628
- **YUAN F. et al.** *eLIFE,* 25 September 2018, vol. 7, e37382
- **DICKINS RA et al.** *Nature Genetics,* 2007, vol. 39 (7), 914-921